(19) Europäisches Patentamt
European Patent Office
Office européen des brevets

(11) EP 2 571 922 B1

(12) # EUROPÄISCHE PATENTSCHRIFT

(45) Veröffentlichungstag und Bekanntmachung des Hinweises auf die Patenterteilung:
**25.06.2014 Patentblatt 2014/26**

(21) Anmeldenummer: **11719029.8**

(22) Anmeldetag: **13.05.2011**

(51) Int Cl.:
***C08G 65/26*** (2006.01) ***C08G 65/00*** (2006.01)

(86) Internationale Anmeldenummer:
**PCT/EP2011/057739**

(87) Internationale Veröffentlichungsnummer:
**WO 2011/144523 (24.11.2011 Gazette 2011/47)**

(54) **VERFAHREN ZUR HERSTELLUNG VON POLYETHERCARBONATPOLYOLEN**
METHOD FOR PRODUCING POLYETHER CARBONATE POLYOLS
PROCÉDÉ POUR PRODUIRE DES POLYÉTHERCARBONATE POLYOLS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2010 EP 10163170**

(43) Veröffentlichungstag der Anmeldung:
**27.03.2013 Patentblatt 2013/13**

(73) Patentinhaber: **Bayer Intellectual Property GmbH**
**40789 Monheim (DE)**

(72) Erfinder:
- **GÜRTLER, Christoph**
**50676 Köln (DE)**
- **HOFMANN, Jörg**
**47800 Krefeld (DE)**
- **WOLF, Aurel**
**42489 Wülfrath (DE)**
- **GRASSER, Stefan**
**51377 Leverkusen (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**WO-A1-03/106025**
**WO-A1-2006/103213**
**WO-A1-2008/013731**
**US-A- 5 783 513**

EP 2 571 922 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung von Polyethercarbonatpolyolen aus einer oder mehrerer H-funktionellen Startsubstanzen, einem oder mehreren Alkylenoxiden und Kohlendioxid in Gegenwart mindestens eines Doppelmetallcyanid-Katalysators, wobei das zur Herstellung des Doppelmetallcyanid-Katalysators eingesetzte cyanidfreie Metallsalz, Metallcyanidsalz oder beide genannten Salze 0,3 bis 1,8 mol Basenäquivalente (bezogen auf 1 mol des zur Katalysatorsynthese eingesetzten Metallcyanidsalzes) in Form eines alkalischen Metallhydroxids, Metallcärbonats und/oder -Metalloxids, enthält.

Die Herstellung von Polyethercarbonatpolyolen durch katalytische Umsetzung von Alkylenoxiden (Epoxiden) und Kohlendioxid in An- oder Abwesenheit von H-funktionellen Startersubstanzen (Startern) wird seit mehr als 40 Jahren intensiv untersucht (z. B. Inoue et al, Copolymerization of Carbon Dioxide and Epoxide with Organometallic Compounds; Die Makromolekulare Chemie 130, 210-220, 1969). Diese Reaktion z.B. uriter Verwendung einer H-funktionellen Starterverbindung ist in Schema (I) schematisch dargestellt, wobei R für einen organischen Rest wie Alkyl, Alkyaryl oder Aryl steht, der jeweils auch Heteroatome wie beispielsweise O, S, Si usw. enthalten kann, und wobei e und f für eine ganzzahlige Zahl stehen, und wobei das hier im Schema (I) gezeigte Produkt für das Polyethercarbonatpolyol lediglich so verstanden werden soll, dass sich Blöcke mit der gezeigten Struktur im erhaltenen Polyethercarbonatpolyol prinzipiell wiederfinden können, die Reihenfolge, Anzahl und Länge der Blöcke sowie die OH-Funktionalität des Starters kann aber variieren und ist nicht auf das in Schema (I) gezeigte Polyethercarbonatpolyol beschränkt. Diese Reaktion (siehe Schema (I)) ist ökologisch sehr vorteilhaft, da diese Reaktion die Umsetzung eines Treibhausgases wie $CO_2$ zu einem Polymer darstellt. Als weiteres Produkt, eigentlich Nebenprodukt, entsteht das in Formel (I) gezeigte cyclische Carbonat (beispielsweise für R = $CH_3$ Propylencarbonat).

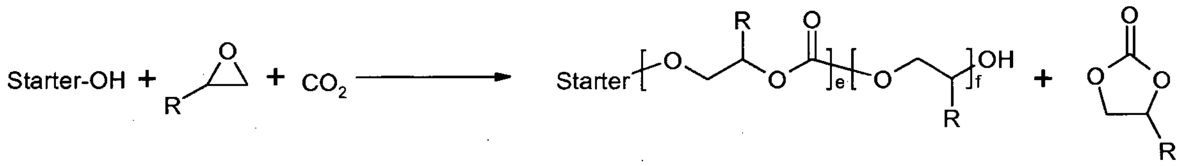
(I)

Als Aktivierung im Sinne der Erfindung wird ein Schritt bezeichnet, bei dem eine Teilmenge Alkylenoxidverbindung, gegebenenfalls in Gegenwart von $CO_2$, zum DMC-Katalysator gegeben wird und dann die Zugabe der Alkylenoxidverbindung unterbrochen wird, wobei aufgrund einer folgenden exothermen chemischen Reaktion eine Wärmeentwicklung, die zu einer Temperaturspitze ("Hotspot") führen kann, sowie aufgrund der Umsetzung von Alkylenoxid und gegebenenfalls $CO_2$ ein Druckabfall im Reaktor beobachtet wird. Der Verfahrensschritt der Aktivierung ist die Zeitspanne von der Zugabe der Teilmenge an Alkylenoxidverbindung, gegebenenfalls in Gegenwart von $CO_2$, zum DMC-Katalysator bis zum Auftreten der Wärmeentwicklung. Im Allgemeinen kann dem Aktivierungsschritt ein Schritt zum Trocknen des DMC-Katalysators und ggf. des Starters bei erhöhter Temperatur und/oder reduziertem Druck vorgelagert sein, wobei dieser Schritt der Trocknung nicht Teil des Aktivierungsschrittes im Sinne der vorliegenden Erfindung ist.

Die Bildung von Copolymeren aus Epoxiden (z.B. Propylenoxid) und Kohlendioxid ist seit langem bekannt. So beschreibt zum Beispiel US 4500704 die Copolymerisation von Kohlendioxid und Propylenoxid unter Verwendung von DMC-Katalysatoren. Hier wurden beispielsweise in einem Reaktor ausgehend von einer Startersubstanz und 12,3 g Propylenoxid (212 mmol) und einem Druck an Kohlendioxid von 48 bar nach 48 Stunden bei 35 °C 71% des Propylenoxids umgesetzt. Von den umgesetzten 150,5 mmol Propylenoxid reagierten 27 mmol (18 %) zum im Allgemeinen unerwünschten Nebenprodukt Propylencarbonat.

WO-A 2006/103213 offenbart ein Verfahren, mit dessen Hilfe die Bildung von cyclischen Carbonaten verringert wird. Dies wird dadurch erreicht, dass zum DMC-Katalysator eine $CO_2$-phile Substanz (z.B. perfluorierte Verbindungen) zugegeben wird. Es werden selbst bei einem hohen $CO_2$-Druck von 62 bar niedrige Propylencarbonat-Gehalte gefunden. So liegt z.B. der Gehalt an Propylencarbonat in Beispiel 6 der Druckschrift bei nur 2,7%, wobei aber die Polydispersität von 7,98 unerwünscht hoch ist.

Da Propylencarbonat einen außerordentlich hohen Siedepunkt von 240 °C bei Normaldruck aufweist, ist dessen Abtrennung aus dem Reaktionsgemisch aufwendig und zeitintensiv. Es ist daher wünschenswert, ein Verfahren zur Copolymerisation von Epoxiden mit Kohlendioxid zu entwickeln, wobei möglichst geringe Mengen an cyclischem Carbonat (wie beispielsweise Propylencarbonat) gebildet werden. Es war daher Aufgabe der vorliegenden Erfindung, ein Verfahren bereitzustellen, mit dem Polyethercarbonatpolyole mit verbesserter Selektivität (d.h. möglichst niedriges Verhältnis von cyclischem Carbonat zu linearem Polyethercarbonat) hergestellt werden können, wobei die Wartezeit ("Zeit 1" in Tabelle 1) bis zur Temperaturspitze bei der Copolymerisation weniger als 120 min betragen soll. Eine kürzere Wartezeit wirkt sich vorteilhaft auf die Wirtschaftlichkeit des Verfahrens aus.

Überraschenderweise wurde nun gefunden, dass ein Verfahren gemäß Anspruch 1 zur Herstellung von Polyethercarbonatpolyolen aus einer oder mehreren H-funktionellen Startsubstanzen, aus einem oder mehreren Alkylenoxiden und Kohlendioxid in Gegenwart mindestens eines DMC-Katalysators die oben genannte Aufgabe löst.

Bei dem zur Herstellung des DMC-Katalysators eingesetzten Metallcyanidsalzes handelt es sich um Kaliumhexacyanocobaltat (III). Unter Basenaquivalente eines alkalischen Metalmydroxids, Metallcarbonats und/oder Metalloxids wird im Sinne der Erfindung verstanden die Äquivalente der resultierenden Hydroxidionen bei einer vollständigen Dissoziation der eingesetzten alkalischen Metallhydroxide, Metallcarbonate und/oder Metalloxide in Wasser. Beispielsweise resultieren aus 0,5 mol eines Metallcarbonats 1,0 mol Basenäquivalente.

Die Summe der eingesetzten alkalischen Metallhydroxide, Metallcarbonate und/oder -Metalloxide beträgt 0,3 bis 1,8 mol, bevorzugt 0,6 bis 1,6 mol, besonders bevorzugt 0,8 bis 1,4 mol Basenäquivalente (bezogen auf 1 mol des zur Katalysatorsynthese eingesetzten Kaliumhexacyanocobaltat (III).

Das Verfahren zur Herstellung von Polyethercarbonatpolyolen aus mindestens einer H-funktionellen Startsubstanz, aus mindestens einem Alkylenoxid und Kohlendioxid in Gegenwart eines DMC-Katalysators kann kontinuierlich, semi-batchartig oder diskontinuierlich durchgeführt werden.

Die erfindungsgemäß erhaltenen Polyethercarbonatpolyole haben im allgemeinen eine Funktionalität von mindestens 1, bevorzugt von 2 bis 8, besonders bevorzugt von 2 bis 6 und ganz besonders bevorzugt von 2 bis 4. Das Molekulargewicht beträgt bevorzugt 400 bis 10000 g/mol und besonders bevorzugt 500 bis 6000 g/mol.

Allgemein können für das erfindungsgemäße Verfahren Alkylenoxide mit 2-24 Kohlenstoffatomen eingesetzt werden. Bei den Alkylenoxiden mit 2-24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinerioxid, ein- oder mehrfach epoxidierte Fette als Mono-, Di- und Triglyceride, epoxidierte Fettsäuren, $C_1$-$C_{24}$-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxy-propyltrimethoxysilan, 3-Glycidyloxypropyltriethoxysilan, 3-Glycidyloxypropyltripropoxysilan, 3-Glycidyloxypropyl-methyl-dimethoxysilan, 3-Glycidyloxypropyl-ethyldiethoxysilan, 3-Glycidyloxypropyltrlisopropoxysilan. Vorzugsweise werden als Alkylenoxide Ethylenoxid und/oder Propylenoxid, insbesondere Propylenoxid eingesetzt.

Als geeignete H-funktionelle Startsubstanz können Verbindungen mit für die Alkoxylierung aktiven H-Atomen eingesetzt werden. Für die Alkoxylierung aktive Gruppen mit aktiven H-Atomen sind beispielsweise -OH, $-NH_2$ (primäre Amine), -NH- (sekundäre Amine), -SH und $-CO_2H$, bevorzugt sind -OH und $-NH_2$ , besonders bevorzugt ist -OH. Als H-funktionelle Startersubstanz wird beispielsweise eine oder mehrere Verbindungen ausgewählt aus der Gruppe bestehend aus ein- oder mehrwertige Alkohole, ein- oder mehrwertige Amine, mehrwertige Thiole, Carbonsäuren, Aminoalkohole, Aminocarbonsäuren, Thioalkohole, Hydroxyester, Polyetherpolyole, Polyesterpolyole, Polyesteretherpolyole, Polyethercarbonatpolyole, Polycarbonatpolyole, Polycarbonate, Polyethylenimine, Polyetheramine (z. B. sogenannte Jeffamine® von Huntsman, wie z. B. D-230, D-400, D-2000, T-403, T-3000, T-5000 oder entsprechende Produkte der BASF, wie z. B. Polyetheramin D230, D400, D200, T403, T5000), Polytetrahydrofurane (z. B. PolyTHF® der BASF, wie z. B. PolyTHF® 250, 650S, 1000, 1000S, 1400, 1800, 2000), Polytetrahydrofuranamine (BASF Produkt Polytetrahydrofuranamin 1700), Polyetherthiole, Polyacrylatpolyole, Ricinusöl, das Mono- oder Diglycerid von Ricinolsäure, Monoglyceride von Fettsäuren, chemisch modifizierte Mono-, Di- und/oder Triglyceride von Fettsäuren, und $C_1$-$C_{24}$ Alkyl-Fettsäureester, die im Mittel mindestens 2 OH-Gruppen pro Molekül enthalten, eingesetzt. Beispielhaft handelt es sich bei den $C_1$-$C_{24}$ Alkyl-Fettsäureester, die im Mittel mindestens 2 OH-Gruppen pro Molekül enthalten, um Handelsprodukte wie Lupranol Balance® (Fa. BASF SE), Merginol®-Typen (Fa. Hobum Oleochemicals GmbH), Sovermol®-Typen (Fa. Cognis Deutschland GmbH & Co. KG) und Soyol®TM-Typen (Fa. USSC Co.).

Als monofunktionelle Startersubstanzen können Alkohole, Amine, Thiole und Carbonsäuren eingesetzt werden. Als monofunktionelle Alkohole können Verwendung finden: Methanol, Ethanol, 1-Propanol, 2-Propanol, 1-Butanol, 2-Butanol, tert-Butanol, 3-Buten-1-ol, 3-Butin-1-ol, 2-Methyl-3-buten-2-o 1, 2-Methyl-3-butin-2-ol, Propagylalkohol, 2-Methyl-2-propanol, 1-tert-Butoxy-2-propanol., 1-Pentanol, 2-Pentanol, 3-Pentanol, 1-Hexanol, 2-Hexanol, 3-Hexanol, 1-Heptanol, 2-Heptanol, 3-Heptanol, 1-Octanol, 2-Octanol, 3-Octanol, 4-Octanol, Phenol, 2-Hydroxybiphenyl, 3-Hydroxybiphenyl, 4-Hydroxybiphenyl, 2-Hydroxypyridin, 3-Hydroxypyridin, 4-Hydroxypyridin. Als monofunktionelle Amine kommen in Frage: Butylamin, tert-Butylamin, Pentylamin, Hexylamin, Anilin, Aziridin, Pyrrolidin, Piperidin, Morpholin. Als monofunktionelle Thiole können verwendet werden: Ethanthiol, 1-Propanthiol, 2-Propanthiol, 1-Butanthiol, 3-Methyl-1-butanthiol, 2-Buten-1-thiol, Thiophenol. Als monofunktionelle Carbonsäuren seien genannt: Ameisensäure, Essigsäure, Propionsäure, Buttersäure, Fettsäuren wie Stearinsäure, Palmitinsäure, Ölsäure, Linolsäure, Linolensäure, Benzoesäure, Acrylsäure.

Als H-funktionelle Startersubstanzen geeignete mehrwertige Alkohole sind beispielweise zweiwertige Alkohole

(wie beispielweise Ethylenglykol, Diethylenglykol, Propylenglykol, Dipropylenglykol, 1,3-Propandiol, 1,4-Butandiol, 1,4-Butendiol, 1,4-Butindiol, Neopentylglykol, 1,5-Pentantandiol, Methylpentandiole (wie beispielweise 3-Methyl-1,5-pentandiol), 1,6-Hexandiol; 1,8-Octandiol, 1,10-Decandiol, 1,12-Dodecandiol, Bis-(hydroxymethyl)-cyclohexane (wie beispielweise 1,4-Bis-(hydroxymethyl)cyclohexan), Triethylenglykol, Tetraethylenglykol, Polyethylenglykole, Dipropylenglykol, Tripropylenglykol, Polypropylenglykole, Dibutylenglykol und Polybutylenglykole); dreiwertige Alkohole (wie beispielweise Trimethylolpropan, Glycerin, Trishydroxyethylisocyanurat, Rizinusöl); vierwertige Alkohole (wie beispielsweise Pentaerythrit); Polyalkohole (wie beispielweise Sorbit, Hexit, Saccharose, Stärke, Stärkehydrolysate, Cellulose, Cellulosehydrolysate, hydroxyfunktionalisierte Fette und Öle, insbesondere Rizinusöl), sowie alle Modifizierungsprodukte dieser zuvor genannten Alkohole mit unterschiedlichen Mengen an ε- Caprolacton.

Die H-funktionellen Startersubstanzen können auch aus der Substanzklasse der Polyetherpolyole ausgewählt sein, insbesondere solchen mit einem Molekulargewicht Mn im Bereich von 100 bis 4000 g/mol. Bevorzugt sind Polyetherpolyole, die aus sich wiederholenden Ethylenoxid- und Propylenoxideinheiten aufgebaut sind, bevorzugt mit einem Anteil von 35 bis 100% Propylenoxideinheiten, besonders bevorzugt mit einem Anteil von 50 bis 100% Propylenoxideinheiten. Hierbei kann es sich um statistische Copolymere, Gradienten-Copolymere, alternierende oder Blockcopolymere aus Ethylenoxid und Propylenoxid handeln. Geeignete Polyetherpolyole, aufgebaut aus sich wiederholenden Propylenoxid- und/oder Ethylenoxideinheiten sind beispielsweise die Desmophen®-, Acclaim®-, Arcol®-, Baycoll®-, Bayfill®-, Bayflex®- Baygal®-, PET^E- und Polyether-Polyole der Bayer MaterialScience AG (wie z. B. Desmophen® 3600Z, Desmophen® 1900U, Acclaim® Polyol 2200, Acclaim® Polyol 4000I, Arcol® Polyol 1004, Arcol® Polyol 1010, Arcol® Polyol 1030, Arcol® Polyol 1070, Baycoll® BD 1110, Bayfill® VPPU 0789, Baygal® K55, PET® 1004, Polyether® S180). Weitere geeignete homo-Polyethylenoxide sind beispielsweise die Pluriol® E-Marken der BASF SE, geeignete homo-Polypropylenoxide sind beispielsweise die Pluriol® P-Marken der BASF SE, geeignete gemischte Copolymere aus Ethylenoxid und Propylenoxid sind beispielsweise die Pluronic® PE oder Pluriol® RPE-Marken der BASF SE.

Die H-funktionellen Startersubstanzen können auch aus der Substanzklasse der Polyesterpolyole ausgewählt sein, insbesondere solchen mit einem Molekulargewicht Mn im Bereich von 200 bis 4500 g/mol. Als Polyesterpolyole werden mindestens difunktionelle Polyester eingesetzt. Bevorzugt bestehen Polyesterpolyole aus alternierenden Säure- und Alkoholeinheiten. Als Säurekomponenten werden z. B. Bernsteinsäure, Maleinsäure, Maleinsäureanhydrid, Adipinsäure, Phthalsäureanhydrid, Phthalsäure, Isophthalsäure, Terephthalsäure, Tetrahydrophtalsäure, Tetrahydrophthalsäureanhydrid, Hexahydrophthalsäureanhydrid oder Gemische aus den genannten Säuren und/oder Anhydriden eingesetzt. Als Alkoholkomponenten werden z. B. Ethandiol, 1,2-Propandiol, 1,3-Propandiol, 1,4-Butandiol, 1,5-Pentandiol, Neopentylglykol, 1,6-Hexandiol, 1,4-Bis-(hydroxymethyl)-cyclohexan, Diethylenglykol, Dipropylenglykol, Trimethylolpropan, Glycerin, Pentaerythrit oder Gemische aus den genannten Alkoholen verwendet. Werden als Alkoholkomponente zweiwertige oder mehrwertige Polyetherpolyole eingesetzt, so erhält man Polyesteretherpolyole die ebenfalls als Startersubstanzen zur Herstellung der Polyethercarbonatpolyole dienen können. Bevorzugt werden Polyetherpolyole mit Mn = 150 bis 2000 g/mol zur Herstellung der Polyesteretherpolyole eingesetzt.

Des weiteren können als H-funktionelle Startersubstanzen Polycarbonatdiole eingesetzt werden, insbesondere solchen mit einem Molekulargewicht Mn im Bereich von 150 bis 4500 g/mol, vorzugsweise 500 bis 2500, die beispielsweise durch Umsetzung von Phosgen, Dimethylcarbonat, Diethylcarbonat oder Diphenylcarbonat und difunktionellen Alkoholen oder Polyesterpolyolen oder Polyetherpolyolen hergestellt werden. Beispiele zu Polycarbonaten finden sich z. B. in der EP-A 1359177. Beispielsweise können als Polycarbonatdiole die Desmophen® C-Typen der Bayer MaterialScience AG verwendet werden, wie z. B. Desmophen® C 1100 oder Desmophen® C 2200.

In einer weiteren Ausführungsform der Erfindung können Polyethercarbonatpolyole als H-funktionelle Startersubstanzen eingesetzt werden. Insbesondere werden Polyethercarbonatpolyole, die nach dem hier beschriebenen erfindungsgemäßen Verfahren erhältlich sind, eingesetzt. Diese als H-funktionelle Startersubstanzen eingesetzten Polyethercarbonatpolyole werden hierzu in einem separaten Reaktionsschritt zuvor hergestellt.

Die H-funktionellen Startersubstanzen weisen im Allgemeinen eine Funktionalität (d.h. Anzahl an für die Polymerisation aktiven H-Atome pro Molekül) von 1 bis 8, bevorzugt von 2 oder 3 auf. Die H-funktionellen Startersubstanzen werden entweder einzeln oder als Gemisch aus mindestens zwei H-funktionellen Startersubstanzen eingesetzt.

Bevorzugte H-funktionelle Startersubstanzen sind Alkohole der allgemeinen Formel (II),

$$HO\text{-}(CH_2)_x\text{-}OH \qquad (II)$$

wobei x eine Zahl von 1 bis 20, bevorzugt eine gerade Zahl von 2 bis 20 ist. Beispiele für Alkohole gemäß Formel (II) sind Ethylenglycol, 1,4-Butandiol, 1,6-Hexandiol, 1,8-Octandiol, 1,10 Decandiol und 1,12-Dodecandiol. Weitere bevorzugte H-funktionelle Startersubstanzen sind Neopentylglykol, Trimethylolpropan, Glycerin, Pentaerythrit, Umsetzungsprodukte der Alkohole gemäß Formel (II) mit ε-Caprolacton, z.B. Umsetzungsprodukte von Trimethylolpropan mit ε-Caprolacton, Umsetzungsprodukte von Glycerin mit ε-Caprolacton, sowie Umsetzungsprodukte von Pentaerythrit mit ε-Caprolacton. Weiterhin bevorzugt werden als H-funktionelle Startsubstanzen Diethylenglykol, Dipropylenglykol, Rizinusöl, Sorbit und Polyetherpolyole, aufgebaut aus sich wiederholenden Polyalkylenoxideinheiten, eingesetzt.

Besonders bevorzugt handelt es sich bei den H-funktionellen Startersubstanzen um eine oder mehrere Verbindungen ausgewählt aus der Gruppe bestehend aus Ethylenglykol, Propylenglykol, 1,3-Propandiol, 1,3-Butandiol, 1,4-Butandiol, 1,5-Pentandiol, 2-Methylpropan-1,3-diol, Neopentylglykol, 1,6-Hexandiol, 1,8 Octandiol, Diethylenglykol, Dipropylenglykol, Glycerin, Trimethylolpropan, di- und trifunktionelle Polyetherpolyole, wobei das Polyetherpolyol aus einer di-oder tri-H-funktionellen Startersubstanz und Propylenoxid bzw. einer di- oder tri-H-funktionellen Startersubstanz, Propylenoxid und Ethylenoxid aufgebaut ist. Die Polyetherpolyole haben bevorzugt ein Molekulargewicht Mn im Bereich von 62 bis 4500 g/mol und eine Funktionalität von 2 bis 3 und insbesondere ein Molekulargewicht Mn im Bereich von 62 bis 3000 g/mol und eine Funktionalität von 2 bis 3.

Die Herstellung der Polyethercarbonatpolyole erfolgt durch katalytische Anlagerung von Kohlendioxid und Alkylenoxiden an H-funktionelle Startersubstanzen. Unter "H-funktionell" wird im Sinne der Erfindung die Anzahl an für die Alkoxylierung aktiven H-Atomen pro Molekül der Startersubstanz verstanden.

DMC-Katalysatoren sind im Prinzip aus dem Stand der Technik bekannt (siehe z.B. US-A 3 404 109, US-A 3 829 505, US-A 3 941 849 und US-A 5 158 922). DMC-Katalysatoren, die z.B. in US-A 5 470 813, EP-A 700 949, EP-A 743 093, EP-A 761 708, WO 97/40086, WO 98/16310 und WO 00/47649 beschrieben sind, besitzen eine sehr hohe Aktivität in der Homopolymerisation von Epoxiden und ermöglichen die Herstellung von Polyetherpolyolen bei sehr geringen Katalysatorkonzentrationen (25 ppm oder weniger), so dass eine Abtrennung des Katalysators aus dem fertigen Produkt i.a. nicht mehr erforderlich ist. Ein typisches Beispiel sind die in EP-A 700 949 beschriebenen hochaktiven DMC-Katalysatoren, die neben einer Doppelmetallcyanid-Verbindung (z.B. Zinkhexacyanocobaltat(III)) und einem organischen Komplexliganden (z.B. tert.-Butanol) noch einen Polyether mit einem zahlenmittlerem Molekulargewicht größer als 500 g/mol enthalten.

Die Herstellung von alkalischen DMC-Katalysatoren wurde in US 5 783 513 offengelegt. Gegenstand der beschriebenen Erfindung waren DMC-Katalysatoren mit einer Alkalinität im Bereich von 0,2 bis etwa 2,0 Gew.-% als Metalloxid basierend auf der Masse Metallsalz, die zur Herstellung des Katalysators verwendet wurde. Diese substantiell nicht kristallinen Katalysatoren führten zu einer verbesserten Viskosität und einem geringeren Grad an Ungesättigtheit bei der Herstellung von Polyetherpolyolen aus Alkylenoxiden. Die Katalysatoren wurden also nur in der Homo-Polymerisation von Propylenoxid eingesetzt. Hinweise auf eine Co-Polymerisation unter Verwendung von Kohlendioxid finden sich in der Schrift nicht.

US 6 716 788 B2 offenbart die Herstellung von alkalischen DMC-Katalysatoren in Gegenwart von 0,03 bis 0,4 mol einer alkalischen Metallverbindung (Zugabe von Oxiden und/oder Hydroxiden ) bezogen auf die Menge eingesetztes Metallsalz, das mit Metallcyanidsalz zur Reaktion gebracht wird. Die so hergestellten DMC-Katalysatoren werden in der Homo-Polymerisation von Epoxiden in Abwesenheit von Kohlendioxid eingesetzt. Hinweise auf eine Co-Polymerisation unter Verwendung von Kohlendioxid finden sich nicht.

In einem bevorzugten Verfahren der vorliegenden Erfindung werden die DMC-Katalysatoren erhalten, indem man

(i) im ersten Schritt eine wässrige Lösung eines cyanidfreien Metallsalzes mit der wässrigen Lösung von Kaliumhexacyanoncobalt (III) in Gegenwart eines oder mehrerer organischen Komplexliganden, z.B. eines Ethers oder Alkohols, umsetzt, wobei ein oder mehrere alkalische Metallhydroxide, Metallcarbonate und/oder -Metalloxide entweder in der wässrigen Lösung des cyanidfreien Metallsalzes, der wässrigen Lösung des Kaliumhexacyanocobaltats (III) oder in beiden wässrigen Lösungen enthalten ist und wobei die Summe der eingesetzten alkalischen Metallhydroxide, Metallcarbonate und/oder -Metalloxide 0,3 bis 1,8 mol, bevorzugt 0,6 bis 1,6 mol, besonders bevorzugt 0,8 bis 1,4 mol Basenäquivalente (bezogen auf 1 mol des zur Katalysatorsynthese eingesetzten Metallcyanidsalzes) beträgt,

(ii) wobei im zweiten Schritt der Feststoff aus der aus (i) erhaltenen Suspension durch bekannte Techniken (wie Zentrifugation oder Filtration) abgetrennt wird,

(iii) wobei gegebenenfalls in einem dritten Schritt der isolierte Feststoff mit einer wässrigen Lösung eines organischen Komplexliganden gewaschen wird (z.B. durch Resuspendieren und anschließende erneute Isolierung durch Filtration oder Zentrifugation),

(iv) wobei anschließend der erhaltene Feststoff, gegebenenfalls nach Pulverisierung, bei Temperaturen von im allgemeinen 20 - 100°C und bei Drücken von im Allgemeinen 0,1 mbar bis Normaldruck (1013 mbar) getrocknet wird, und wobei im ersten Schritt oder unmittelbar nach der Ausfällung der Doppelmetallcyanidverbindung (zweiter Schritt) ein oder mehrere organische Komplexliganden, vorzugsweise im Überschuß (bezogen auf die Doppelmetallcyanidverbindung) und gegebenenfalls weitere komplexbildende Komponenten zugesetzt werden.

Die in den erfindungsgemäß verwendeten DMC-Katalysatoren enthaltenen Doppelmetallcyanid-Verbindungen sind die Reaktionsprodukte wasserlöslicher cyanidfreier Metallsalze und Kaliumhexacyanoncobaltat (III), wobei das zur Herstellung des DMC-Katalysators eingesetzte cyanidfreie Metallsalz, Kaliumhexacyanocobaltat (III) oder beide genannten Salze 0,3 bis 1,8 mol, bevorzugt 0,6 bis 1,6 mol, besonders bevorzugt 0,8 bis 1,4 mol Basenäquivalente (bezogen

auf 1 mol des zur Katalysatorsynthese eingesetzten Kaliumhexacyanocobaltat (III)) in Form eines alkalisches Metallhydroxids, Metallcarbonats und/oder -Metalloxids, enthält.

Beispielsweise wird eine wässrige Lösung von Zinkchlorid (bevorzugt im Überschuß bezogen auf das Kaliumhexacyanocobaltat) und Kaliumhexacyanocobaltat gemischt und anschließend Dimethoxyethan (Glyme) oder *tert*-Butanol (vorzugsweise im Überschuß, bezogen auf Zinkhexacyanocobaltat) zur gebildeten Suspension gegeben, wobei das eingesetzte Kaliumhexacyanocobaltat zuvor mit 0,3 bis 1,8 mol, bevorzugt 0,6 bis 1,6 mol, besonders bevorzugt g 0,8 bis 4 mol Basenäquivalente (bezogen auf 1 mol des zur Katalysatorsynthese eingesetzten Kaliumhexacyanocobaltat) an alkalischem Metallhydroxid, Metallcarbonat und/oder -Metalloxid vermischt wurde.

Zur Herstellung der Doppelmetallcyanid-Verbindungen geeignete cyanidfreie Metallsalze besitzen bevorzugt die allgemeine Formel (III),

$$M(X)_n \qquad (III)$$

wobei

M ausgewählt ist aus den Metallkationen $Zn^{2+}$, $Fe^{2+}$, $Ni^{2+}$, $Mn^{2+}$, $Co^{2+}$, $Sr^{2+}$, $Sn^{2+}$, $Pb^{2+}$ und, $Cu^{2+}$, bevorzugt ist M $Zn^{2+}$, $Fe^{2+}$, $Co^{2+}$ oder $Ni^{2+}$, X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

n ist 1, wenn X = Sulfat, Carbonat oder Oxalat ist und

n ist 2, wenn X = Halogenid, Hydroxid, Cyanat, Thiocyanat, Isocyanat, Isothiocyanat oder Nitrat ist,

oder geeignete cyanidfreie Metallsalze besitzen die allgemeine Formel (IV),

$$M_r(X)_3 \qquad (IV)$$

wobei

M ausgewählt ist aus den Metallkationen $Fe^{3+}$, $Al^{3+}$ und $Cr^{3+}$,

X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

r ist 2, wenn X = Sulfat, Carbonat oder Oxalat ist und

r ist 1, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (V),

$$M(X)_s \qquad (V)$$

wobei

M ausgewählt ist aus den Metallkationen $Mo^{4+}$, $V^{4+}$ und $W^{4+}$

X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat, Thiocyanat, Isocyanat, Isothiocyanat, Carboxylat, Oxalat und Nitrat;

s ist 2, wenn X = Sulfat, Carbonat oder Oxalat ist und

s ist 4, wenn X = Halogenid, Hydroxid, Cyanat, Thiocyanat, Isocyanat, Isothiocyanat, Carboxylat oder Nitrat ist,

oder geeignete cyanidfreie Metallsalze besitzen die allgemeine Formel (VI),

$$M(X)_t \qquad (VI)$$

wobei

M ausgewählt ist aus den Metallkationen $Mo^{6+}$ und $W^{6+}$

X sind ein oder mehrere (d.h.verschiedene) Anionen, vorzugsweise ein Anion ausgewählt aus der Gruppe der Halogenide (d.h. Fluorid, Chlorid, Bromid, Iodid), Hydroxid, Sulfat, Carbonat, Cyanat,

Thiocyanat, Isocyanat, Isothiocyanat, Carboxylat, Oxalat und Nitrat; 5 t ist 3, wenn X = Sulfat, Carbonat oder Oxalat ist und

t ist 6, wenn X = Halogenid, Hydroxid, Cyanat, Thiocyanat, Isocyanat, Isothiocyanat, Carboxylat oder Nitrat ist,

Beispiele geeigneter cyanidfreier Metallsalze sind Zinkchlorid, Zinkbromid, Zinkjodid, Zinkacetat, 0 Zinkacetylacetonat, Zinkbenzoat, Zinknitrat, Eisen(II)sulfat, Eisen(II)bromid, Eisen(II)chlorid, Cobalt(iiiiii)chlorid, Cobalt(II)thiocyanat, Nickel(II)chlorid und Nickel(II)nitrat. Es können auch Mischungen verschiedener Metallsalze eingesetzt werden.

Das zur Herstellung der Doppelmetallcyanid-Verbindungen verwendete Metallcyanidsalz ist Kaliumhexacyanocobaltat(III). Bevorzugte Doppelmetallcyanid-Verbindungen, die in den erfindungsgemäßen DMC-Katalysatoren enthalten sind, sind Verbindungen der allgemeinen Formel (VIII)

$M_xLM'_{x'}(CN)_y]_z$ (VIII),

worin M wie in Formel (III) bis (VI) und
M' Co (III) ist, und
x, x', y und z sind ganzzahlig und so gewählt, dass die Elektronenneutralität der Doppelmetallcyanidverbindung gegeben ist.
Vorzugsweise ist
=3,x'=l,y=6undz=2,
M = Zn(II), Fe(II), Co(II) oder Ni(II).

Beispiele geeigneter Doppelmetallcyanid-Verbindungen sind Zinkhexacyanocobaltat (III), und Cobalt(II)hexacyanocobaltat(III).

Die zur Herstellung der erfindungsgemäß eingesetzten DMC-Katalysatoren eingesetzten alkalischen Metallhydroxide, -carbonate und -oxide sind vorzugsweise die Oxide oder Hydroxide von Metallen der Gruppen 1a und 2a des Periodensystems der Elemente (siehe beispielsweise "Handbook of Chemistry and Physics, 63rd Edition"). Beispiele geeigneter alkalischer Metallhydroxide, Matalloxide und -Metallcarbonate sind Natriumcarbonat, Natriumhydroxid, Kaliumcarbonat, Kaliumhydroxid, Calciumoxid, Calciumhydroxid, Bariumhydroxid oder Bariumoxid.

Die bei der Herstellung der DMC-Katalysatoren zugesetzten organischen Komplexliganden sind beispielsweise in US 5 158 922 (siehe insbesondere Spalte 6, Zeilen 9 bis 65), US 3 404 109, US 3 829 505, US 3 941 849, EP-A 700 949, EP-A 761 708, JP 4 145 123, US 5 470 813, EP-A 743 093 und WO-A 97/40086) offenbart. Beispielsweise werden als organische Komplexliganden wasserlösliche, organische Verbindungen mit Heteroatomen, wie Sauerstoff, Stickstoff, Phosphor oder Schwefel, die mit der Doppelmetallcyanid-Verbindung Komplexe bilden können, eingesetzt. Bevorzugte organische Komplexliganden sind Alkohole, Aldehyde, Ketone, Ether, Ester, Amide, Harnstoffe, Nitrile, Sulfide und deren Mischungen. Besonders bevorzugte organische Komplexliganden sind aliphatische Ether (wie Dimethoxyethan), wasserlösliche aliphatische Alkohole (wie Ethanol, Isopropanol, n-Butanol, iso-Butanol, sek.-Butanol, tert-Butanol, 2-Methyl-3-buten-2-ol und 2-Methyl-3-butin-2-ol), Verbindungen, die sowohl aliphatische oder cycloaliphatische Ethergruppen wie auch aliphatische Hydroxylgruppen enthalten (wie z.B. Ethylenglykol-mono-tert.-butylether, Diethylenglykol-mono-tert.-butylether, Tripropylenglykol-mono-methylether und 3-Methyl-3-oxetan-methanol). Höchst bevorzugte organische Komplexliganden sind ausgewählt aus einer oder mehrerer Verbindungen der Gruppe bestehend aus Dimethoxyethan, tert-Butanol, 2-Methyl-3-buten-2-ol, 2-Methyl-3-butin-2-ol, Ethylenglykol-mono-tert.-butylether und 3-Methyl-3-oxetan-methanol.

Optional werden bei der Herstellung der erfindungsgemäßen DMC-Katalysatoren eine oder mehrere komplexbildende Komponente(n) aus den Verbindungsklassen der Polyether, Polyester, Polycarbonate, Polyalkylenglykolsorbitanester, Polyalkylenglykolglycidylether, Polyacrylamid, Poly(acrylamid-co-acrylsäure), Polyacrylsäure, Poly(acrylsäure-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol), Oxazolinpolymere, Polyalkylenimine, Maleinsäure- und Maleinsäureanhydridcopolymere, Hydroxyethylcellulose und Polyacetale, oder der Glycidylether, Glycoside, Carbonsäureester mehrwertiger Alkohole, Gallensäuren oder deren Salze, Ester oder Amide, Cyclodextrine, Phosphorverbindungen, $\alpha,\beta$-ungesättigten Carbonsäureester oder ionische oberflächen- bzw. grenzflächenaktiven Verbindungen eingesetzt.

Bevorzugt werden bei der Herstellung der erfindungsgemäßen DMC-Katalysatoren im ersten Schritt die wässrigen Lösungen des Metallsalzes (z.B. Zinkchlorid), eingesetzt im stöchiometrischen Überschuss (mindestens 50 Mol-%) bezogen auf Kaliumhexacyanocabaltat (III), also mindestens ein molares Verhältnis von cyanidfreiem Metallsalz zu Kaliumhexacyanocabaltat (III) von 2,25 zu 1,00.) und des Kaliumhexacyanocobaltat (III) in Gegenwart des organischen Komplexliganden (z.B. tert.-Butanol) umgesetzt, wobei ein oder mehrere alkalische Metallhydroxide, Metallcarbonate und/oder -Metalloxide entweder in der wässrigen Lösung des cyanidfreien Metallsalzes, der wässrigen Lösung des Kaliumhexacyanocobaltat (III) oder in beiden wässrigen Lösungen enthalten ist , so dass sich eine Suspension bildet, die die Doppelmetallcyanid-Verbindung (z.B. Zinkhexacyanocobaltat), Wasser, überschüssiges cyanidfreies Metallsalz, und den organischen Komplexliganden enthält.

Der organische Komplexligand kann dabei in der wässrigen Lösung des cyanidfreien Metallsalzes und/oder des Kaliumhexacyanocobaltat (III) vorhanden sein, oder er wird der nach Ausfällung der Doppelmetallcyanid-Verbindung erhaltenen Suspension unmittelbar zugegeben. Es hat sich als vorteilhaft erwiesen, die wässrigen Lösungen des cyanidfreien Metallsalzes und des Kaliumhexacyanocobaltat (III), wobei ein oder mehrere alkalische Metallhydroxide, Metallcarbonate und/oder -Metalloxide entweder in der wässrigen Lösung des cyanidfreien Metallsalzes, der wässrigen Lösung des Kaliumhexacyanocobaltat (III) oder in beiden wässrigen Lösungen enthalten ist, und den organischen Komplexliganden unter starkem Rühren zu vermischen. Optional wird die im ersten Schritt gebildete Suspension anschließend mit einer weiteren komplexbildenden Komponente behandelt. Die komplexbildende Komponente wird dabei bevorzugt in einer Mischung mit Wasser und organischem Komplexliganden eingesetzt. Ein bevorzugtes Verfahren zur

Durchführung des ersten Schrittes (d.h. der Herstellung der Suspension) erfolgt unter Einsatz einer Mischdüse, besonders bevorzugt unter Einsatz eines Strahldispergators wie in WO-A 01/39883 beschrieben.

Im zweiten Schritt erfolgt die Isolierung des Feststoffs (d.h. die Vorstufe des erfindungsgemäßen Katalysators) aus der Suspension durch bekannte Techniken, wie Zentrifugation oder Filtration.

In einer bevorzugten Ausführungsvariante wird der isolierte Feststoff anschließend in einem dritten Verfahrensschritt mit einer wässrigen Lösung des organischen Komplexliganden gewaschen (z.B. durch Resuspendieren und anschließende erneute Isolierung durch Filtration oder Zentrifugation). Auf diese Weise können zum Beispiel wasserlösliche Nebenprodukte, wie Kaliumchlorid, aus dem erfindungsgemäßen Katalysator entfernt werden. Bevorzugt liegt die Menge des organischen Komplexliganden in der wässrigen Waschlösung zwischen 40 und 80 Gew.-%, bezogen auf die Gesamtlösung.

Optional wird im dritten Schritt der wässrigen Waschlösung weitere komplexbildende Komponente, bevorzugt im Bereich zwischen 0,5 und 5 Gew.-%, bezogen auf die Gesamtlösung, zugefügt.

Außerdem ist es vorteilhaft, den isolierten Feststoff mehr als einmal zu waschen..Vorzugsweise wird in einem ersten Waschschritt (iii-1) mit einer wässrigen Lösung des ungesättigten Alkohols gewaschen (z.B. durch Resuspendieren und anschließende erneute Isolierung durch Filtration oder Zentrifugation), u m auf diese Weise zum Beispiel wasserlösliche Nebenprodukte, wie Kaliumchlorid, aus dem erfindungsgemäßen Katalysator zu entfernen. Besonders bevorzugt liegt die Menge des ungesättigten Alkohols in der wässrigen Waschlösung zwischen 40 und 80 Gew.-%, bezogen auf die Gesamtlösung des ersten Waschschritts. In den weiteren Waschschritten (iii-2) wird entweder der erste Waschschritt einmal oder mehrmals, vorzugsweise einmal bis dreimal wiederholt, oder vorzugsweise wird eine nicht wässrige Lösungen, wie z.B. eine Mischung oder Lösung aus organischem Komplexliganden und weiterer komplexbildender Komponente (bevorzugt im Bereich zwischen 0,5 und 5 Gew.-%, bezogen auf die Gesamtmenge der Waschlösung des Schrittes (iii-2)), als Waschlösung eingesetzt und der Feststoff damit einmal oder mehrmals, vorzugsweise einmal bis dreimal gewaschen.

Der isolierte und gegebenenfalls gewaschene Feststoff wird anschließend, gegebenenfalls nach Pulverisierung, bei Temperaturen von im allgemeinen 20 - 100°C und bei Drücken von im allgemeinen 0,1 mbar bis Normaldruck (1013 mbar) getrocknet.

Ein bevorzugtes Verfahren zur Isolierung der erfindungsgemäßen DMC-Katalysatoren aus der Suspension durch Filtration, Filterkuchenwäsche und Trocknung wird in WO-A 01/80994 beschrieben.

Gegenstand der Erfindung ist auch ein Verfahren zur Herstellung von Polyethercarbonatpolyolen aus einer oder mehrerer H-funktionellen Startsubstanzen, eines oder mehrerer Alkylenoxiden und Kohlendioxid in Gegenwart mindestens eines DMC-Katalysators, wobei das zur Herstellung des DMC-Katalysators eingesetzte cyanidfreie Metallsalz, Kaliumhexacyanocobaltat (III) oder beide genannten Salze 0,3 bis 1,8 mol, bevorzugt 0,6 bis 1,6 mol, besonders bevorzugt 0,8 bis 1,4 mol Basenäquivalente (bezogen auf 1 mol des zur Katalysatorsynthese eingesetzten Kaliumhexacyanocobaltat (III)) in Form eines alkalischen Metallhydroxids, Metallcarbonats und/oder Metalloxids enthält, , und wobei

($\alpha$) die H-funktionelle Startersubstanz oder ein Gemisch aus mindestens zwei H-funktionellen Startersubstanzen vorgelegt und gegebenenfalls Wasser und/oder andere leicht flüchtige Verbindungen durch erhöhte Temperatur und/oder reduziertem Druck entfernt werden ("Trocknung"), wobei der DMC-Katalysator der H-funktionellen Startersubstanz oder dem Gemisch von mindestens zwei H-funktionellen Startersubstanzen vor oder nach der Trocknung zugesetzt wird,

($\beta$) zur Aktivierung

($\beta$1) in einem ersten Aktivierungsschritt eine erste Teilmenge (bezogen auf die Gesamtmenge der bei der Aktivierung und Copolymerisation eingesetzten Menge an Alkylenoxiden) von einem oder mehreren Alkylenoxiden zu der aus Schritt ($\alpha$) resultierenden Mischung zugesetzt wird, wobei diese Zugabe der Teilmenge an Alkylenoxid gegebenenfalls in Gegenwart von $CO_2$ erfolgen kann, vorzugsweise aber unter Abwesenheit von $CO_2$, und wobei dann die aufgrund der folgenden exothermen chemischen Reaktion auftretende Temperaturspitze ("Hotspot") und/oder ein Druckabfall im Reaktor jeweils abgewartet wird,

($\beta$2) in einem zweiten Aktivierungsschritt nach der im vorangegangenen Aktivierungsschritt erreichten Temperaturspritze eine zweite Teilmenge (bezogen auf die Gesamtmenge der bei der Aktivierung und Copolymerisation eingesetzten Menge an Alkylenoxiden) von einem oder mehreren Alkylenoxiden zu der aus dem vorangegangenen Aktivierungsschritt resultierenden Mischung zugesetzt wird, wobei diese Zugabe der Teilmenge an Alkylenoxid gegebenenfalls in Gegenwart von $CO_2$ erfolgen kann, vorzugsweise aber unter Abwesenheit von $CO_2$, und wobei dann die aufgrund der folgenden exothermen chemischen Reaktion auftretende Temperaturspitze ("Hotspot") und/oder ein Druckabfall im Reaktor jeweils abgewartet wird,

(β3) gegebenenfalls in einem dritten Aktivierungsschritt bzw. weiteren Aktivierungsschritten nach der im vorangegangenen Aktivierungsschritt erreichten Temperaturspitze der Schritt (β2) null bis fünfmal, bevorzugt ein bis viermal, besonders bevorzugt genau einmal wiederholt wird, wobei diese Zugabe der Teilmenge bzw. Zugaben der Teilmengen an Alkylenoxid unter Abwesenheit von $CO_2$ erfolgt, und wobei dann die aufgrund der folgenden exothermen chemischen Reaktion auftretende Temperaturspitze ("Hotspot") und/oder ein Druckabfall im Reaktor jeweils abgewartet wird,

(β4) gegebenenfalls in einem weiteren Aktivierungsschritt bzw. weiteren Aktivierungsschritten nach der im vorangegangenen Aktivierungsschritt erreichten Temperaturspitze der Schritt (β3) ein bis fünfmal, bevorzugt ein bis viermal, besonders bevorzugt genau einmal wiederholt wird, wobei diese Zugabe der Teilmenge bzw. Zugaben der Teilmengen an Alkylenoxid in Gegenwart von $CO_2$ erfolgt, und wobei dann die aufgrund der folgenden exothermen chemischen Reaktion auftretende Temperaturspitze ("Hotspot") und/oder ein Druckabfall im Reaktor jeweils abgewartet wird,

(γ) ein oder mehrere Alkylenoxide und Kohlendioxid zu der aus Schritt (β) resultierenden Mischung fortwährend dosiert werden ("Copolymerisation"). Die für die Copolymerisation eingesetzten Alkylenoxide können gleich oder verschieden sein von den bei der Aktivierung eingesetzten Alkylenoxide.

In einer bevorzugten Ausführungsform beträgt die bei der Aktivierung in den Schritten β1 bis β4 jeweils eingesetzte Teilmenge von einem oder mehreren Alkylenoxiden 2,0 bis 15,0 Gew.-%, bevorzugt 2,5 bis 14,0 Gew.-%, besonders bevorzugt 3,0 bis 13,0 Gew.-% (bezogen auf die Gesamtmenge der bei der Aktivierung und Copolymerisation eingesetzten Menge an Alkylenoxiden).

Zu Schritt (α):

Zur Herstellung von Polyethercarbonatpolyolen durch katalytische Anlagerung von Alkylenoxiden (Epoxiden) und Kohlendioxid an H-funktionelle Startersubstanzen (Starter) in Gegenwart der erfindungsgemäßen DMC-Katalysatoren wird die H-funktionelle Startersubstanz oder ein Gemisch aus mindestens zwei H-funktionellen Startersubstanzen vorgelegt. Gegebenenfalls wird in Schritt (α)

(α1) die H-funktionelle Startersubstanz oder ein Gemisch aus mindestens zwei H-funktionellen Startersubstanzen vorgelegt und

(α2) die Temperatur der Startersubstanz oder des Gemisches auf 50 bis 200°C, bevorzugt 80 bis 160°C, besonders bevorzugt bei 100 bis 140°C gebracht und/oder der Druck im Reaktor auf weniger als 500 mbar, bevorzugt 5 mbar bis 100 mbar erniedrigt. Dabei kann auch ein Stickstoff-Strom durch den Reaktor geleitet werden.

Dabei kann der DMC-Katalysator bereits in der H-funktionelle Startersubstanz oder dem Gemisch von mindestens zwei H-funktionellen Startersubstanzen vorgelegt sein, es ist jedoch auch möglich, den dann getrockneten DMC-Katalysator erst nach der Trocknung der H-funktionellen Startersubstanz oder dem Gemisch der H-funktionellen Startersubstanzen zuzusetzen. Der DMC-Katalysator kann in fester Form oder als Suspension in einer H-funktionellen Startersubstanz zugegeben werden. Wird der Katalysator als Suspension zugegeben, wird diese bevorzugt vor der Trocknung der H-funktionellen Startersubstanz(en) zugefügt.

Zu Schritt (β):

Die Dosierung eines oder mehrerer Alkylenoxide und gegebenenfalls des Kohlendioxids erfolgt nach der Trocknung einer Startersubstanz oder der Mischung mehrerer Startersubstanzen und der Zugabe des DMC-Katalysators, der vor oder nach der Trocknung der Startersubstanz als Feststoff oder in Form einer Suspension zugegeben wird. Wird der DMC-Katalysator nach der Trocknung der Startersubstanz zugegeben, so sollte dieser DMC-Katalysator vorzugsweise getrocknet sein, beispielsweise in einem analogen Verfahren wie die Trocknung der Startersubstanz. Die Dosierung eines oder mehrerer Alkylenoxide und des Kohlendioxids kann prinzipiell in unterschiedlicher Weise erfolgen. Der Start der Dosierung kann aus dem Vakuum heraus oder bei einem zuvor gewählten Vordruck erfolgen. Der Vordruck wird bevorzugt durch Einleiten eines Inertgases wie beispielsweise Stickstoff eingestellt, wobei der Druck(absolut) zwischen 10 mbar bis 10 bar, vorzugsweise 100 mbar bis 8 bar und bevorzugt 500 mbar bis 6 bar eingestellt wird. In einer besonders bevorzugten Ausführungsform wird das aus Schritt (α) resultierende Gemisch aus einer oder mehrerer Startersubstanzen und dem DMC-Katalysator bei einer Temperatur von 100°C bis 130°C mindestens einmal, vorzugsweise dreimal mit 1,5 bar bis 10 bar (absolut), besonders bevorzugt 3 bar bis 6 bar (absolut) eines Inertgases (Stickstoff oder ein Edelgas wie beispielsweise Argon) beaufschlagt und jeweils unmittelbar anschließend bis innerhalb von 15 min wird der Überdruck auf 1 bar (absolut) reduziert. Alternativ wird in

einer ebenfalls besonders bevorzugten Ausführungsform in das aus Schritt (α) resultierende Gemisch aus einer oder mehrerern Starterverbindungen und dem DMC-Katalysator bei einer Temperatur von 40°C bis 150°C Inertgas (Stickstoff oder ein Edelgas wie beispielsweise Argon) eingeleitet und gleichzeitig ein reduzierter Druck (absolut) von 10 mbar bis 800 mbar, besonders bevorzugt von 50 mbar bis 200 mbar angelegt.

Zu Schritt (γ):

Die Dosierung eines oder mehrerer Alkylenoxide und des Kohlendioxids kann simultan oder sequentiell erfolgen, wobei die gesamte Kohlendioxidmenge auf einmal oder dosiert über die Reaktionszeit zugegeben werden kann. Vorzugsweise erfolgt eine Dosierung des Kohlendioxids. Die Dosierung eines oder mehrerer Alkylenoxide erfolgt simultan oder sequentiell zur Kohlendioxid Dosierung. Werden mehrere Alkylenoxide zur Synthese der Polyethercarbonatpolyole eingesetzt, so kann deren Dosierung simultan oder sequentiell über jeweils separate Dosierungen erfolgen oder über eine oder mehrere Dosierungen, wobei mindestens zwei Alkylenoxide als Gemisch dosiert werden. Über die Art der Dosierung der Alkylenoxide und des Kohlendioxids ist es möglich, statistische, alternierende, blockartige oder gradientenartige Polyethercarbonatpolyole zu synthetisieren. Die Konzentration an freien Alkylenoxiden während der Reaktion in der Reaktionsmischung beträgt vorzugsweise > 0 bis 40 Gew.-%, besonders bevorzugt > 0 - 25 Gew-%, höchst bevorzugt > 0 - 15 Gew.-% (jeweils bezogen auf das Gewicht der Reaktionsmischung).

Vorzugsweise wird ein Überschuss an Kohlendioxid bezogen auf die berechnete Menge an eingebautem Kohlendioxid im Polyethercarbonatpolyol eingesetzt, d a bedingt durch die Reaktionsträgheit von Kohlendioxid ein Überschuss von Kohlendioxid von Vorteil ist. Die Menge an Kohlendioxid kann über den Gesamtdruck bei den jeweiligen Reaktionsbedingungen festgelegt werden. Als Gesamtdruck (absolut) hat sich der Bereich von 0,01 bis 120 bar, bevorzugt 0,1 bis 110 bar, besonders bevorzugt von 1 bis 100 bar für die Copolymerisation zur Herstellung der Polyethercarbonatpolyole als vorteilhaft erwiesen. Für das erfindungsgemäße Verfahren hat sich weiterhin gezeigt, dass die Copolymerisation zur Herstellung der Polyethercarbonatpolyole vorteilhafterweise bei 50 bis 150°C, bevorzugt bei 60 bis 145°C, besonders bevorzugt bei 70 bis 140°C und ganz besonders bevorzugt bei 110 bis 120°C durchgeführt wird. Werden Temperaturen unterhalb von 50°C eingestellt, kommt die Reaktion zum Erliegen. Bei Temperaturen oberhalb von 150°C steigt die Menge an unerwünschten Nebenprodukten stark an. Weiterhin ist zu beachten, dass das $CO_2$ bei der Wahl von Druck und Temperatur vom gasförmigen Zustand möglichst in den flüssigen und/oder überkritischen flüssigen Zustand übergeht. $CO_2$ kann jedoch auch als Feststoff in den Reaktor gegeben werden und dann unter den gewählten Reaktionsbedingungen in den flüssigen und/oder überkritischen flüssigen Zustand übergehen.

Besonders bevorzugte Reaktoren sind: Rohrreaktor, Rührkessel, Schlaufenreaktor. Polyetherpolycarbonatpolyole können in einem Rührkessel hergestellt werden, wobei der Rührkessel je nach Ausführungsform und Betriebsweise über den Reaktormantel, innenliegende und/oder in einem Umpumpkreislauf befindliche Kühlflächen gekühlt wird. Aus Sicherheitsgründen sollte der Gehalt an freiem Epoxid 15 Gew.-% im Reaktionsgemisch des Rührkessels nicht übersteigen (siehe beispielsweise WO-A 2004/081082; Seite 3; Zeile 14). Sowohl in der semi-batch Anwendung, wo das Produkt erst nach Ende der Reaktion entnommen wird, als auch in der kontinuierlichen Anwendung, wo das Produkt kontinuierlich entnommen wird, ist daher besonders auf die Dosiergeschwindigkeit des Epoxids zu achten. Sie ist so einzustellen, dass trotz der inhibierenden Wirkung des Kohlendioxids das Epoxid genügend schnell abreagiert. Es ist möglich, das Kohlendioxid kontinuierlich oder diskontinuierlich zuzuführen. Dies hängt davon ab, ob das Epoxid schnell genug verbraucht wird und ob das Produkt gegebenenfalls $CO_2$-freie Polyether-Blöcke enthalten soll. Die Menge des Kohlendioxids (angegeben als Druck) kann bei der Zugabe des Epoxids ebenso variieren. Es ist möglich, während der Zugabe des Epoxids den $CO_2$-Druck allmählich zu steigern oder zu senken oder gleich zu lassen.

Eine weitere mögliche Ausführungsform im Rührkessel für die Copolymerisation (Schritt γ) ist dadurch gekennzeichnet, dass eine oder mehrere H-funktionellen Starterverbindungen während der Reaktion kontinuierlich in den Reaktor zudosiert werden. Die Menge der H-funktionellen Starterverbindungen, die während der Reaktion kontinuierlich in den Reaktor zudosiert werden, beträgt bevorzugt mindestens 20 mol% Äquivalente, besonders bevorzugt 70 bis 95 mol% Äquivalente (jeweils bezogen auf die gesamte Menge an H-funktionellen Starterverbindungen).

Die aktivierte Katalysator-Starter-Mischung kann im Rührkessel, aber auch in einem anderen Reaktionsbehältnis (Rohrreaktor oder Schlaufenreaktor) mit Epoxid und Kohlendioxid (weiter) copolymerisiert werden.

Beim Rohrreaktor werden aktivierter Katalysator und Starter sowie Epoxid und Kohlendioxid kontinuierlich durch ein Rohr gepumpt. Die molaren Verhältnisse der Reaktionspartner variieren je nach gewünschtem Polymer. In einer bevorzugten Ausführungsform wird hierbei Kohlendioxid in seiner überkritischen Form, also quasi flüssig, zudosiert, um eine bessere Mischbarkeit der Komponenten zu ermöglichen. Vorteilhafterweise werden Mischelemente zur besseren Durchmischung der Reaktionspartner eingebaut, wie sie zum Beispiel von der Firma Ehrfeld Mikrotechnik BTS GmbH vertrieben werden, oder Mischer-Wärmetauscherelemente, die gleichzeitig die Durchmischung und Wärmeabfuhr verbessern.

Selbst Schlaufenreaktoren können zur Herstellung von Polyetherpolycarbonatpolyolen verwendet werden. Hierunter fallen im Allgemeinen Reaktoren mit Stoffrückführung, wie beispielsweise ein Strahlschlaufenreaktor, der auch kontinuierlich betrieben werden kann, oder eine Schlaufe von Rohrreaktoren. Der Einsatz eines Schlaufenreaktors ist insbesondere deshalb von Vorteil, weil hier eine Rückvermischung realisiert werden kann, so dass die Epoxid-Konzentration gering sein sollte. Um vollständigen Umsatz zu realisieren, ist häufig ein Rohr ("Verweilrohr") nachgeschaltet.

Die nach dem erfindungsgemäßen Verfahren erhältlichen Polyethercarbonatpolyole weisen einen geringen Gehalt an Nebenprodukten auf und können problemlos verarbeitet werden, insbesondere durch Umsetzung mit Di- und/oder Polyisocyanaten zu Polyurethanen, insbesondere Polyurethan-Weichschaumstoffen. Für Polyurethananwendungen werden vorzugsweise Polyethercarbonatpolyole eingesetzt, die auf einer H-funktionellen Startverbindung basieren, welche eine Funktionalität von mindestens 2 besitzt. Des Weiteren können die nach dem erfindungsgemäßen Verfahren erhältlichen Polyethercarbonatpolyole in Anwendungen wie Wasch- und Reinigungsmittelformulierungen, Bohrflüssigkeiten, Kraftstoffadditiven, ionischen und nicht-ionischen Tensiden, Schmiermitteln, Prozesschemikalien für die Papier- oder Textilherstellung oder kosmetischen Formulierungen verwendet werden. Dem Fachmann ist bekannt, dass abhängig vom jeweiligen Anwendungsgebiet die zu verwendenden Polyethercarbonatpolyole gewisse Stoffeigenschaften wie beispielsweise Molekulargewicht, Viskosität, Polydispersität, Funktionalität und/oder Hydroxylzahl erfüllen müssen.

## Beispiele

Das Gewichts- und Zahlenmittel des Molekulargewichts der entstandenen Polymere wurde mittels Gelpermeations-Chromatographie (GPC) bestimmt. Es wurde vorgegangen nach DIN 55672-1: "Gelpermeationschromatographie,Teil - Tetrahydrofuran als Elutionsmittel". Dabei wurden Polystyrolproben bekannter Molmasse zur Kalibrierung verwendet.

Die OH-Zahl (Hydroxylzahl) wurde in Anlehnung an DIN 53240-2 bestimmt, wobei jedoch Pyridin anstelle von THF/Dichlormethan als Lösungsmittel verwendet wurde. Es wurde mit 0,5 molarer ethanolischer KOH titriert (Endpunktserkennung mittels Potentiometrie). Als Prüfsubstanz fungierte Rizinusöl mit durch Zertifikat festgelegter OH-Zahl. Die Angabe der Einheit in "mg/g" bezieht sich auf mg[KOH]/g[Polyethercarbonatpolyol].

Der Anteil an eingebautem $CO_2$, im resultierenden Polyethercarbonatpolyol sowie das Verhältnis von Propylencarbonat zu Polyethercarbonatpolyol wurden mittels $^1$-NMR (Firma Bruker, DPX 400, 400 MHz; Pulsprogramm zg30, Wartezeit d1: 10s, 64 Scans) bestimmt. Die Probe wurde jeweils in deuteriertem Chloroform gelöst. Die relevanten Resonanzen im $^1$H-NMR (bezogen auf TMS = 0 ppm) sind wie folgt:

cyclisches Carbonat (welches als Nebenprodukt gebildet wurde) Resonanz bei 4,5 ppm, Carbonat, resultierend aus im Polyethercarbonatpolyol eingebautem Kohlendioxid (Resonanzen bei 5,1 bis 4,8 ppm), nicht abreagiertes PO mit Resonanz bei 2,4 ppm, Polyetherpolyol (d.h. ohne eingebautem Kohlendioxid) mit Resonanzen bei 1,2 bis 1,0 ppm, das als Startermolekül eingebaute 1,8 Octandiol mit einer Resonanz bei 1,6 bis 1,52 ppm.

Der Molenanteil des im Polymer eingebauten Carbonats in der Reaktionsmischung wird nach Formel (IX) wie folgt berechnet, wobei folgende Abkürzungen verwendet werden:

F(4,5) = Fläche der Resonanz bei 4,5 ppm für cyclisches Carbonat (entspricht einem H Atom)
F(5,1-4,8) = Fläche der Resonanz bei 5,1-4,8 ppm für Polyethercarbonatpolyol und einem H-Atom für cyclisches Carbonat.
F(2,4) = Fläche der Resonanz bei 2,4 ppm für freies, nicht abreagiertes PO
F(1,2-1,0) = Fläche der Resonanz bei 1,2-1,0 ppm für Polyetherpolyol
F(1,6-1,52) = Fläche der Resonanz bei 1,6 bis 1,52 ppm für 1,8 Octandiol (Starter)

Unter Berücksichtigung der relativen Intensitäten wurde gemäß der folgenden Formel (IX) für das polymer gebundene Carbonat ("lineares Carbonat" LC) in der Reaktionsmischung in mol% umgerechnet:

$$LC = \frac{F(5{,}1-4{,}8) - F(4{,}5)}{F(5{,}1-4{,}8) + F(2{,}4) + 0{,}33 * F(1{,}2-1{,}0) + 0{,}25 * F(1{,}6-1{,}52)} * 100 \qquad (IX)$$

Der Gewichtsanteil (in Gew.-%) polymer-gebundenen Carbonats (LC') in der Reaktionsmischung wurde nach Formel (X) berechnet,

$$LC' = \frac{[F(5,1-4,8) - F(4,5)] * 102}{N} * 100\% \qquad (X)$$

wobei sich der Wert für N ("Nenner" N) nach Formel (XI) berechnet:

N=[F(5,1-4,8)-F(4,5)]*102+F(4,5)*102+F(2,4)*58+0,33*F(1,2-1,0)*58+0,25*F(1,6-1,52)*146 (XI)

Der Faktor 102 resultiert aus der Summe der Molmassen von $CO_2$ (Molmasse 44 g/mol) und der von Propylenoxid (Molmasse 58 g/mol), der Faktor 58 resultiert aus der Molmasse von Propylenoxid und der Faktor 146 resultiert aus der Molmasse des eingesetzten Starters 1,8-Octandiol.

Der Gewichtsanteil (in Gew.-%) an cyclischem Carbonat (CC') in der Reaktionsmischung wurde nach Formel (XII) berechnet,

$$CC' = \frac{F(4,5) * 102}{N} * 100\% \qquad (XII)$$

wobei sich der Wert für N nach Formel (XI) berechnet.

Um aus den Werten der Zusammensetzung der Reaktionsmischung die Zusammensetzung bezogen auf den Polymer-Anteil (bestehend aus Polyetherpolyol, welches aus Starter und Propylenoxid während der unter $CO_2$-freien Bedingungen stattfindenden Aktivierungsschritte aufgebaut wurde, und Polyethercarbonatpolyol, aufgebaut aus Starter, Propylenoxid und Kohlendioxid während der in Gegenwart von $CO_2$ stattfindenden Aktivierungsschritten und während der Copolymerisation) zu berechnen, wurden die Nicht-Polymer-Bestandteile der Reaktionsmischung (d.h. cyclisches Propylencarbonat sowie ggf. vorhandenes, nicht umgesetztes Propylenoxid) rechnerisch eliminiert. Der Gewichtsanteil der Carbonat-Wiederholungseinheiten im Polyethercarbonatpolyol wurde in einen Gewichtsanteil Kohlendioxid mittels des Faktors F=44/(44+58) umgerechnet. Die Angabe des $CO_2$-Gehalts im Polyethercarbonatpolyol ("eingebautes $CO_2$"; siehe nachfolgende Beispiele und Tabelle 1) ist normiert auf den Anteil des Polyethercarbonatpolyol-Moleküls, das bei der Copolymerisation und ggf. den Aktivierungsschritten in Gegenwart von $CO_2$ gebildet wurde (d.h. der Anteil des Polyethercarbonatpolyol-Moleküls, der aus dem Starter (1,8-Octandiol) sowie aus der Reaktion des Starters mit Epoxid resultiert, das unter $CO_2$-freien Bedingungen zugegeben wurde, wurde hierbei nicht berücksichtigt).

Eingesetzte H-funktionelle Starterverbindung:

1,8-Octandiol Fa. Sigma Aldrich

<u>Beispiele 1 bis 7: Die Katalysatoren wurden wie folgt hergestellt:</u>

Beispiel 1 (Vergleich): Herstellung eines DMC-Katalysators ohne Zugabe von NaOH

Der Katalysator wurde mit einer Apparatur gemäß Fig. 4 aus WO-A 01/39883 hergestellt. In einem Schlaufenreaktor, der einen Strahldispergator gemäß Fig. 2 aus WO-A 01/39883 mit einer Bohrung (Durchmesser 0,7 mm) enthält, wurde eine Lösung aus 258 g Zinkchlorid in 937 g destilliertem Wasser und 135 g tert.-Butanol bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert.

230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiel 2 (Vergleich): Herstellung eines DMC-Katalysators mit 0,25 mol Basenäquivalenten pro mol Kaliumhexacyanocobaltat

Der Katalysator wurde mit einer Apparatur gemäß Fig. 4 aus WO-A 01/39883 hergestellt.

In einem Schlaufenreaktor, der einen Strahldispergator gemäß Fig. 2 aus WO-A 01/39883 mit einer Bohrung (Durchmesser 0,7 mm) enthält, wurde eine Lösung aus 258 g Zinkchlorid in 937 g destilliertem Wasser, 135 g tert.-Butanol

und 7,8 g einer 10%-igen wässrigen NaOH (0,0195 mol Basenäquivalente) bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert.

230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiel 3: Herstellung eines DMC-Katalysators mit 0,55 mol Basenäquivalenten pro mol Kaliumhexacyanocobaltat

In einem 1 1 Rundkolben wurde eine Lösung aus 7,4 g (0,022 mol) Kaliumhexacyanocobaltat, 39 g tert.-Butanol und 302 g dest. Wasser vorgelegt und unter starkem Rühren mittels eines Laborrührers (1000 U/min) auf 30°C aufgeheizt. Zur gerührten Lösung wurden innerhalb von 3 min 152 g einer 50%-igen wässrigen Lösung von Zinkchlorid (Alkalinität 0,64 Gew.-% ZnO, entspricht 0,012 mol Baseäquivalenten)) zugetropft. Anschließend wurde weitere 30 min bei 30°C gerührt (1000 U/min). Die gebildete Suspension wurde mittels einer Filternutsche filtriert. Anschließend wurden 8,0 g des feuchten Filterkuchens unter starkem Rühren (1000 U/min) in einer Mischung aus 110 g tert.-Butanol und 60 g dest. Wasser dispergiert. Nachdem der komplette Feststoff in der Waschlösung homogen dispergiert war, wurde noch weitere 30 min gerührt. Die Suspension wurde wieder mittels einer Filternutsche filtriert und der feuchte Filterkuchen abschließend noch einmal in 144 g tert.-Butanol dispergiert. Nach Filtration der Dispersion wurde der Filterkuchen bei 45°C über Nacht im Vakuum (300 mbar) getrocknet.

Beispiel 4: Herstellung eines DMC-Katalysators mit 0,372 mol Basenäquivalenten pro mol Kaliumhexacyanocobaltat

Der Katalysator wurde mit einer Apparatur gemäß Fig. 4 aus WO-A 01/39883 hergestellt.

In einem Schlaufenreaktor, der einen Strahldispergator gemäß Fig. 2 aus WO-A 01/39883 mit einer Bohrung (Durchmesser 0,7 mm) enthält, wurde eine Lösung aus 258 g Zinkchlorid in 937 g destilliertem Wasser, 135 g tert.-Butanol und 15,3 g einer 10%-igen wässrigen NatriumcarbonatLösung (0,0145 mol) bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert. 230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiel 5 (Vergleich): Herstellung eines DMC-Katalysators mit 0,20 mol Basenäquivalenten pro mol Kaliumhexacyanocobaltat

Der Katalysator wurde mit einer Apparatur gemäß Fig. 4 aus WO-A 01/39883 hergestellt.

In einem Schlaufenreaktor, der einen Strahldispergator gemäß Fig. 2 aus WO-A 01/39883 mit einer Bohrung (Durchmesser 0,7 mm) enthält, wurde eine Lösung aus 258 g Zinkchlorid in 937 g destilliertem Wasser, 135 g tert.-Butanol und 15,3 g einer 10%-igen wässrigen Natriummonomethylcarbonat-Lösung (0,0156 mol) bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert.

230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiel 6: Herstellung eines DMC-Katalysators mit 1,25 mol Basenäquivalenten pro mol Kaliumhexacyanocobaltat

Der Katalysator wurde mit einer Apparatur gemäß Fig. 4 aus WO-A 01/39883 hergestellt.

In einem Schlaufenreaktor, der einen Strahldispergator gemäß Fig. 2 aus WO-A 01/39883 mit einer Bohrung (Durchmesser 0,7 mm) enthält, wurde eine Lösung aus 427 g Zinkbromid in 937 g destilliertem Wasser, 135 g tert.-Butanol und 39,0 g einer 10%-igen wässrigen NaOH (0,0975 mol) bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert.

230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiel 7 (Vergleich): Herstellung eines DMC-Katalysators mit 2,0 mol Basenäquivalenten pro mol Kaliumhexacyanocobaltat

Der Katalysator wurde mit einer Apparatur gemäß Fig. 4 aus WO-A 01/39883 hergestellt.

In einem Schlaufenreaktor, der einen Strahldispergator gemäß Fig. 2 aus WO-A 01/39883 mit einer Bohrung (Durchmesser 0,7 mm) enthält, wurde eine Lösung aus 427 g Zinkbromid in 937 g destilliertem Wasser, 135 g tert.-Butanol und 62,4 g einer 10%-igen wässrigen NaOH (0,156 mol) bei 50°C zirkuliert. Hierzu wurde eine Lösung aus 26 g Kaliumhexacyanocobaltat (0,078 mol) in 332 g destilliertem Wasser zudosiert. Der Druckverlust im Strahldispergator betrug dabei 2,5 bar. Anschließend wurde die gebildete Dispersion 60 min bei 50°C und einem Druckverlust im Strahldispergator von 2,5 bar zirkuliert. Danach wurde eine Mischung aus 5,7 g tert.-Butanol, 159 g destilliertem Wasser und 27,6 g Polypropylenglykol 1000 zudosiert und die Dispersion dann 80 min bei 50°C und einem Druckverlust im Strahldispergator von 2,5 bar zirkuliert.

230 g der erhaltenen Dispersion wurden in einer Drucknutsche mit 20 $cm^3$ Filterfläche filtriert und anschließend mit einer Mischung aus 82 g tert.-Butanol, 42,3 g destilliertem Wasser und 1,7 g Polypropylenglykol 1000 gewaschen. Der gewaschene Filterkuchen wurde mechanisch zwischen 2 Streifen Filterpapier abgepresst und abschließend für 2 h bei 60°C im Hochvakuum bei ca. 0,05 bar (absolut) getrocknet.

Beispiele 8 bis 14: Die Katalysatoren wurden in der Herstellung von Polyethercarbonatpolyolen wie folgt getestet:

In einen 1 Liter Druckreaktor mit Gasdosierungseinrichtung wurden 141 mg getrockneter DMC-Katalysator gemäß einem der Beispiele 1 bis 7 (siehe nachfolgende Tabelle 1) sowie getrocknetes 51 g 1,8-Octandiol (Starter) vorgelegt. Der Reaktor wurde auf 130 °C aufgeheizt und durch wiederholtes Beaufschlagen von Stickstoff auf ca. 5 bar und nachfolgendes Entspannen auf ca. 1 bar inertisiert. Dieser Vorgang wurde 3-mal durchgeführt In den Reaktor wurden bei 130 °C und Abwesenheit von $CO_2$ 25 g Propylenoxid (PO) schnell eindosiert. Die Aktivierung des Katalysators machte sich durch eine Temperaturspitze ("Hotspot") und durch Druckabfall auf den Ausgangsdruck (ca. 1 bar) bemerkbar. Nach dem ersten Druckabfall wurden 20 g PO und dann 19 g PO schnell eindosiert, wodurch es wiederum jeweils zu einer Temperaturspitze und zu einem Druckabfall kam. Nachdem der Reaktor mit 50 bar $CO_2$ beaufschlagt worden war, wurden 50 g PO schnell eindosiert, wodurch es nach einer Wartezeit [**Zeit 1**] zu einer Temperaturspitze kam. Gleichzeitig dazu fing der Druck an Kohlendioxid $CO_2$ zu fallen an. Der Druck wurde so geregelt, dass bei Absinken unter den Sollwert neues $CO_2$ zugegeben wurde. Erst danach wurde das restliche Propylenoxid (435 g) kontinuierlich mit ca. 1,8 g/min in den Reaktor gepumpt, während nach 10 Minuten in Schritten von 5°C pro fünf Minuten die Temperatur auf 105 °C gesenkt wurde. Nach beendeter PO-Zugabe wurde noch solange bei 105 °C und dem oben angegebenen Druck weitergerührt (1500 U/min), bis kein Verbrauch an $CO_2$ mehr beobachtet wurde.

Tabelle 1: Herstellung von Polyethercarbonatpolyolen

| Beispiel | Eingesetzter Katalysator aus Beispiel | Alkalinität [mol Basenäquiva. pro mol $K_3[Co(CN)_6]$] | Zeit 1 [min] | Eingebautes $CO_2$ [Gew.-%] | Selektivität cyclisch / linear | OH-Zahl [mg/g] | Polydispersität |
|---|---|---|---|---|---|---|---|
| **8 (Vgl)** | 1 (Vgl) | -- | 30 | 21,0 | 0,20 | 65,5 | 1,70 |
| **9 (Vgl)** | 2 (Vgl) | 0,25 | 38 | 21,0 | 0,21 | 64,6 | 1,52 |

(fortgesetzt)

| Beispiel | Eingesetzter Katalysator aus Beispiel | Alkalinität [mol Basenäquiva. pro mol $K_3[Co(CN)_6]$] | Zeit 1 [min] | Eingebautes $CO_2$ [Gew.-%] | Selektivität cyclisch / linear | OH-Zahl [mg/g] | Polydispersität |
|---|---|---|---|---|---|---|---|
| **10** | 3 | 0,55 | 74 | 19,3 | 0,16 | 66,6 | 1,68 |
| **11** | 4 | 0,372 | 58 | 18,7 | 0,17 | 65,3 | 1,52 |
| **12 (Vgl)** | 5 (Vgl) | 0,20 | 114 | 22,4 | 0,22 | 75,2 | 1,91 |
| **13** | 6 | 1,25 | 50 | 18,8 | 0,14 | 48,7 * | 1,44 |
| **14 (Vgl)** | 7 (Vgl) | 2,0 | 188 | 18,5 | 0,14 | 57,3 * | 1,59 |

Vgl = Vergleichsbeispiel
*) OH-Zahl der Reaktionsmischung, wobei gebildetes Propylencarbonat nicht vorher abgetrennt wurde

Es wird aus den Ergebnissen der Tabelle 1 deutlich, dass eine Mindestmenge an Base notwendig ist, um eine Verbesserung der Selektivität relativ zu einem DMC-Katalysator ohne Basenzusatz zu erzielen. So wird die Selektivität ohne Basenzusatz (Bsp. 8 (Vgl)) durch Zugabe von 0,2 (Bsp. 12 (Vgl) oder 0,25 (Bsp. 9 (Vgl)) Basenäquivalenten pro mol $K_3[Co(CN)_6]$ bei der Herstellung des DMC-Katalysators nicht verbessert. Erst ab einer Alkalinität von mehr als 0,25 Basenäquivalenten pro mol $K_3[Co(CN)_6]$, z.B. 0,37 Basenäquivalenten pro mol $K_3[Co(CN)_6]$ gemäß Bsp. 11, wird die Selektivität verbessert zugunsten des gewünschten linearen Polyethercarbonatpolyols. Ein zu hoher Anteil an Basenäquivalenten pro mol $K_3[Co(CN)_6]$ verschlechtert dagegen die Wirtschaftlichkeit des Verfahrens; bereits ein Anteil an von 2,0 mol Basenäquivalenten pro mol $K_3[Co(CN)_6]$ verlängert die Zeit 1 auf über 120 min (Bsp. 14 (Vgl.)).

## Patentansprüche

1. Verfahren zur Herstellung von Polyethercarbonatpolyolen aus einer oder mehreren H-funktionellen Startsubstanzen, aus einem oder mehreren Alkylenoxiden und Kohlendioxid in Gegenwart mindestens eines Doppelmetallcyanid-Katalysators,
**dadurch gekennzeichnet, dass** der DMC-Katalysator hergestellt wird, indem eine wässrige Lösung eines cyanidfreien Metallsalzes mit der wässrigen Lösung von Kaliumhexacyanocobaltat(III) in Gegenwart eines oder mehrerer organischen Komplexliganden umgesetzt wird, wobei ein oder mehrere alkalische Metallhydroxide, Metallcarbonate und/oder -Metalloxide entweder in der wässrigen Lösung des cyanidfreien Metallsalzes, der wässrigen Lösung des Kaliumhexacyanocobaltats(III) oder in beiden wässrigen Lösungen enthalten sind, und wobei die Summe der eingesetzten alkalischen Metallhydroxide, Metallcarbonate und/oder -Metalloxide 0,3 bis 1,8 mol Basenäquivalente (bezogen auf 1 mol des zur Katalysatorsynthese eingesetzten Kaliumhexacyanocobaltat(III)) beträgt.

2. Verfahren gemäß Anspruch 1, wobei der DMC-Katalysator hergestellt wird, indem

(i) im ersten Schritt eine wässrige Lösung eines cyanidfreien Metallsalzes mit der wässrigen Lösung an Kaliumhexacyanocobaltat (III) in Gegenwart eines oder mehrerer organischen Komplexliganden umsetzt, wobei ein oder mehrere alkalische Metallhydroxide und/oder Metallcarbonate und/oder -Metalloxide entweder in der wässrigen Lösung des cyanidfreien Metallsalzes, der wässrigen Lösung des Kalium¬hexa¬cyano¬cobaltat(III) oder in beiden wässrigen Lösungen enthalten ist und wobei die Summe der eingesetzten alkalischen Metallhydroxide und/oder
Metallcarbonate und/oder Metalloxide 0,3 bis 1,8 mol Basenäquivalente (bezogen auf 1 mol des zur Katalysatorsynthese eingesetzten Kaliumhexacyanocobaltat (III) beträgt,
(ii) wobei im zweiten Schritt der Feststoffs aus der aus (i) erhaltenen Suspension abgetrennt wird,
(iii) wobei in einem dritten Schritt der isolierte Feststoff mit einer wässrigen Lösung eines organischen Komplexliganden gewaschen wird,
(iv) wobei anschließend der erhaltene Feststoff getrocknet wird,
und wobei im ersten Schritt oder unmittelbar nach der Ausfällung der Doppelmetallcyanidverbindung (zweiter Schritt) ein oder mehrere organische Komplexliganden, zugesetzt werden.

3. Verfahren gemäß Anspruch 1 oder 2, wobei das zur Herstellung des Doppelmetallcyanid-Katalysators eingesetzte cyanidfreie Metallsalz, Kaliumhexacyanocobaltat (III) oder beide genannten Salze 0,6 bis 1,6 mol Basenäquivalente

(bezogen auf 1 mol des zur Katalysatorsynthese eingesetzten Kaliumhexacyanocobaltat (III) in Form eines alkalischen Metallhydroxids, Metallcarbonats und/oder Metalloxids, enthält.

4. Verfahren gemäß einem der Ansprüche 1 bis 3, wobei das eingesetzte alkalische Metallhydroxid, Metallcarbonat und/oder -Metalloxid ausgewählt ist aus mindestens einem der Gruppe bestehend aus den Oxide oder Hydroxiden von Metallen der Gruppen 1a und 2a des Periodensystems der Elemente.

5. Verfahren gemäß Anspruch 4, wobei das eingesetzte alkalische Metallhydroxid, Metalloxid und/oder -Metallcarbonat ausgewählt ist aus mindestens einem der Gruppe bestehend aus Natriumcarbonat, Natriumhydroxid, Kaliumhydroxid, Kaliumcarbonat, Calciumoxid, Calciumhydroxid, Bariumhydroxid und Bariumoxid

6. Verfahren gemäß Anspruch 2, wobei das eingesetzte cyanidfreie Metallsalz zur Bildung der DMC-Verbindung ausgewählt ist aus mindestens einem der Gruppe bestehend aus Zinkchlorid, Zinkbromid, Zinkjodid, Zinkacetat, Zinkacetylacetonat, Zinkbenzoat, Zinknitrat, Eisen(II)sulfat, Eisen(II)bromid, Eisen(II)chlorid, Cobalt(II)chlorid, Cobalt(II)thiocyanat, Nickel(II)chlorid und Nickel(II)nitrat.

7. Verfahren gemäß einem der Ansprüche 1 bis 6, **dadurch gekennzeichnet, dass**

(α) die H-funktionelle Startersubstanz oder ein Gemisch aus mindestens zwei H-funktionellen Startersubstanzen vorgelegt und gegebenenfalls Wasser und/oder andere leicht flüchtige Verbindungen durch erhöhte Temperatur und/oder reduziertem Druck entfernt werden ("Trocknung"), wobei der DMC-Katalysator der H-funktionellen Startersubstanz oder dem Gemisch von mindestens zwei H-funktionellen Startersubstanzen vor oder nach der Trocknung zugesetzt wird,
(β) zur Aktivierung

(β1) in einem ersten Aktivierungsschritt eine erste Teilmenge (bezogen auf die Gesamtmenge der bei der Aktivierung und Copolymerisation eingesetzten Menge an Alkylenoxiden) von einem oder mehreren Alkylenoxiden zu der aus Schritt (α) resultierenden Mischung zugesetzt wird, und wobei dann die aufgrund der folgenden exothermen chemischen Reaktion auftretende Temperaturspitze ("Hotspot") und/oder ein Druckabfall im Reaktor jeweils abgewartet wird,
(β2) in einem zweiten Aktivierungsschritt nach der im vorangegangenen Aktivierungsschritt erreichten Temperaturspritze eine zweite Teilmenge (bezogen auf die Gesamtmenge der bei der Aktivierung und Copolymerisation eingesetzten Menge an Alkylenoxiden) von einem oder mehreren Alkylenoxiden zu der aus dem vorangegangenen Aktivierungsschritt resultierenden Mischung zugesetzt wird, und wobei dann die aufgrund der folgenden exothermen chemischen Reaktion auftretende Temperaturspitze ("Hotspot") und/oder ein Druckabfall im Reaktor jeweils abgewartet wird,
(γ) ein oder mehrere Alkylenoxide und Kohlendioxid zu der aus Schritt (β) resultierenden Mischung zugesetzt werden ("Copolymerisation").

8. Verfahren gemäß einem der Ansprüche 1 bis 7, **dadurch gekennzeichnet, dass** die Copolymerisation von Alkylenoxiden und Kohlendioxid unter einem Druck von 1 bis 200 bar erfolgt.

9. Verfahren gemäß einem der Ansprüche 1 bis 8, **dadurch gekennzeichnet, dass** die Copolymerisation von Alkylenoxiden und Kohlendioxid (Schritt (γ)) im Temperaturbereich von 60 bis 150 °C erfolgt.

10. Verfahren gemäß einem der Ansprüche 1 bis 9, welches in einem Rohrreaktor, Rührkessel oder Schlaufenreaktor durchgeführt wird.

11. Verfahren gemäß einem der Ansprüche 1 bis 10 durchgeführt in einem Rührkessel, **dadurch gekennzeichnet, dass** in Schritt γ) eine oder mehrere H-funktionellen Starterverbindungen während der Reaktion kontinuierlich in den Reaktor zudosiert werden.

## Claims

1. Process for preparing polyether carbonate polyols from one or more H-functional starter substances, one or more alkylene oxides and carbon dioxide in the presence of at least one double metal cyanide catalyst, **characterized in that** the DMC catalyst is prepared by reacting an aqueous solution of a cyanide-free metal salt with the aqueous

solution of potassium hexacyanocobaltate (III) in the presence of one or more organic complex ligands, one or more alkaline metal hydroxides, metal carbonates and/or metal oxides being present either in the aqueous solution of the cyanide-free metal salt or the aqueous solution of the potassium hexacyanocobaltate (III), or in both aqueous solutions, and the sum of the alkaline metal hydroxides, metal carbonates and/or metal oxides used being 0.3 to 1.8 mol of base equivalents (based on 1 mol of the potassium hexacyanocobaltate (III) used for catalyst synthesis).

**2.** Process according to Claim 1, wherein the DMC catalyst is prepared by

(i) in the first step, reacting an aqueous solution of a cyanide-free metal salt with the aqueous solution of potassium hexacyanocobaltate (III) in the presence of one or more organic complex ligands, one or more alkaline metal hydroxides and/or metal carbonates and/or metal oxides being present either in the aqueous solution of the cyanide-free metal salt or the aqueous solution of the potassium hexacyanocobaltate (III), or in both aqueous solutions, and the sum of the alkaline metal hydroxides and/or metal carbonates and/or metal oxides used being 0.3 to 1.8 mol of base equivalents (based on 1 mol of the potassium hexacyanocohaltate (III) used for catalyst synthesis),
(ii) in the second step, removing the solids from the suspension obtained from (i),
(iii) in a third step, washing the isolated solids with an aqueous solution of an organic complex ligand,
(iv) then drying the resulting solids,
and, in the first step or immediately after the precipitation of the double metal cyanide compound (second step), one or more organic complex ligands are added.

**3.** Process according to Claim 1 or 2, wherein the cyanide-free metal salt or potassium hexacyanocobaltate (III) used for preparation of the double metal cyanide catalyst contains, or both of these salts contain, 0.6 to 1.6 mol of base equivalents (based on 1 mol of the potassium hexacyanocobaltate (III) used for catalyst synthesis) in the form of an alkaline metal hydroxide, metal carbonate and/or metal oxide.

**4.** Process according to any of Claims 1 to 3, wherein the alkaline metal hydroxide, metal carbonate and/or metal oxide used is selected from at least one of the group consisting of the oxides or hydroxides of metals of groups 1a and 2a of the Periodic Table of the Elements.

**5.** Process according to Claim 4, wherein the alkaline metal hydroxide, metal oxide and/or metal carbonate used is selected from at least one of the group consisting of sodium carbonate, sodium hydroxide, potassium hydroxide, potassium carbonate, calcium oxide, calcium hydroxide, barium hydroxide and barium oxide.

**6.** Process according to Claim 2, wherein the cyanide-free metal salt used for formation of the DMC compound is selected from at least one of the group consisting of zinc chloride, zinc bromide, zinc iodide, zinc acetate, zinc acetylacetonate, zinc benzoate, zinc nitrate, iron(II) sulfate, iron(II) bromide, iron(II) chloride, cobalt(II) chloride, cobalt(II) thiocyanate, nickel(II) chloride and nickel(II) nitrate.

**7.** Process according to any of Claims 1 to 6, **characterized in that**

($\alpha$) the H-functional starter substance or a mixture of at least two H-functional starter substances is initially charged and water and/or other volatile compounds are optionally removed by means of elevated temperature and/or reduced pressure ("drying"), the DMC catalyst being added to the H-functional starter substance or to the mixture of at least two H-functional starter substances before or after the drying,
($\beta$) activation is accomplished by

($\beta$1) in a first activation step, adding a first portion (based on the total amount of the amount of alkylene oxides used in the activation and copolymerization) of one or more alkylene oxides to the mixture resulting from step ($\alpha$), and in each case awaiting the temperature peak ("hotspot") which occurs due to the exothermic chemical reaction which follows and/or a pressure drop in the reactor,
($\beta$2) in a second activation step, after the temperature peak attained in the preceding activation step, adding a second portion (based on the total amount of the amount of alkylene oxides used in the activation and copolymerization) of one or more alkylene oxides to the mixture resulting from the preceding activation step, and in each case awaiting the temperature peak ("hotspot") which occurs due to the exothermic chemical reaction which follows and/or a pressure drop in the reactor,
($\gamma$) one or more alkylene oxides and carbon dioxide are added to the mixture resulting from step ($\beta$) ("copolymerization").

**8.** Process according to any of Claims 1 to 7, **characterized in that** the copolymerization of alkylene oxides and carbon dioxide is effected under a pressure of 1 to 200 bar.

**9.** Process according to any of Claims 1 to 8, **characterized in that** the copolymerization of alkylene oxides and carbon dioxide (step (γ)) is effected within the temperature range from 60 to 150°C.

**10.** Process according to any of Claims 1 to 9, which is performed in a tubular reactor, stirred tank or loop reactor.

**11.** Process according to any of Claims 1 to 10 performed in a stirred tank, **characterized in that**, in step γ), one or more H-functional starter compounds are metered continuously into the reactor during the reaction.

## Revendications

**1.** Procédé pour la préparation de polyéthercarbonate-polyols à partir d'une ou de plusieurs substances de départ à fonctionnalité H, d'un ou de plusieurs oxydes d'alkylène et de dioxyde de carbone en présence d'au moins un catalyseur de type cyanure métallique double (DMC), **caractérisé en ce que** le catalyseur DMC est préparé par transformation d'une solution aqueuse d'un sel métallique exempt de cyanure avec la solution aqueuse d'hexacyanocobaltate (III) de potassium en présence d'un ou de plusieurs ligands complexes organiques, un ou plusieurs hydroxydes, carbonates et/ou oxydes métalliques alcalins étant contenu(s) soit dans la solution aqueuse du sel métallique exempt de cyanure, soit dans la solution aqueuse de l'hexacyanocobaltate (III) de potassium ou dans les deux solutions aqueuses et la somme des hydroxydes, carbonates et/ou oxydes métalliques alcalins valant 0,3 à 1,8 mole d'équivalent de base (par rapport à 1 mole de l'hexacyanocobaltate (III) de potassium utilisé pour la synthèse du catalyseur).

**2.** Procédé selon la revendication 1, le catalyseur de DMC étant préparé en ce que

(i) dans une première étape, on transforme une solution aqueuse d'un sel métallique exempt de cyanure avec la solution aqueuse d'hexacyanocobaltate (III) de potassium en présence d'un ou de plusieurs ligands complexes organiques, un ou plusieurs hydroxydes et/ou carbonates et/ou oxydes métalliques alcalins étant contenu(s) soit dans la solution aqueuse du sel métallique exempt de cyanure, soit dans la solution aqueuse de l'hexacyanocobaltate (III) de potassium ou dans les deux solutions aqueuses et la somme des hydroxydes et/ou carbonates et/ou oxydes métalliques alcalins valant 0,3 à 1,8 mole d'équivalent de base (par rapport à 1 mole de l'hexacyanocobaltate (III) de potassium utilisé pour la synthèse du catalyseur),
(ii) dans une deuxième étape, on sépare le solide de la suspension obtenue dans (i),
(iii) dans une troisième étape, on lave le solide isolé avec une solution aqueuse d'un ligand complexe organique,
(iv) on sèche ensuite le solide obtenu,
et en ajoutant, dans la première étape ou juste après la précipitation du composé de type cyanure métallique double (deuxième étape) un ou plusieurs ligands complexes organiques.

**3.** Procédé selon la revendication 1 ou 2, le sel métallique exempt de cyanure, l'hexacyanocobaltate (III) de potassium ou les deux sels, utilisé(s) pour la préparation du catalyseur de type cyanure métallique double contenant 0,6 à 1,6 mole d'équivalent de base (par rapport à 1 mole de l'hexacyanocobaltate (III) de potassium utilisé pour la synthèse du catalyseur) sous forme d'un hydroxyde, d'un carbonate et/ou d'un oxyde métallique alcalin.

**4.** Procédé selon l'une quelconque des revendications 1 à 3, l'hydroxyde, le carbonate et/ou l'oxyde métallique alcalin utilisé étant choisi parmi au moins un composé du groupe constitué par les oxydes ou les hydroxydes de métaux des groupes 1a et 2a du système périodique des éléments.

**5.** Procédé selon la revendication 4, l'hydroxyde, l'oxyde et/ou le carbonate métallique alcalin étant choisi parmi au moins un composé du groupe constitué par le carbonate de sodium, l'hydroxyde de sodium, l'hydroxyde de potassium, le carbonate de potassium, l'oxyde de calcium, l'hydroxyde de calcium, l'hydroxyde de baryum et l'oxyde de baryum.

**6.** Procédé selon la revendication 2, le sel métallique exempt de cyanure utilisé pour la formation du composé DMC étant choisi parmi au moins un sel du groupe constitué par le chlorure de zinc, le bromure de zinc, l'iodure de zinc, l'acétate de zinc, l'acétylacétonate de zinc, le benzoate de zinc, le nitrate de zinc, le sulfate de fer (II), le bromure de fer (II), le chlorure de fer (II), le chlorure de cobalt (II), le thiocyanate de cobalt (II), le chlorure de nickel (II) et le

nitrate de nickel (II).

**7.** Procédé selon l'une quelconque des revendications 1 à 6, **caractérisé en ce que**

($\alpha$) on dispose au préalable la substance de départ à fonctionnalité H ou un mélange d'au moins deux substances de départ à fonctionnalité H et on élimine le cas échéant l'eau et/ou d'autres composés volatils par une température augmentée et/ou une pression réduite ("séchage"), le catalyseur DMC étant ajouté à la substance de départ à fonctionnalité H ou au mélange d'au moins deux substances de départ à fonctionnalité H avant ou après le séchage,
(ß) pour l'activation :

(ß1) dans une première étape d'activation, on ajoute une première quantité partielle (par rapport à la quantité totale de la quantité d'oxydes d'alkylène utilisée lors de l'activation et de la copolymérisation) d'un ou de plusieurs oxydes d'alkylène au mélange résultant de l'étape ($\alpha$) et en attendant ensuite à chaque fois le pic de température ("hotspot") et/ou une chute de pression dans le réacteur, survenant en raison de la réaction chimique exothermique qui suit,
(ß2) dans une deuxième étape d'activation, après le pic de température atteint dans l'étape d'activation précédente, on ajoute une deuxième quantité partielle (par rapport à la quantité totale de la quantité d'oxydes d'alkylène utilisée lors de l'activation et de la copolymérisation) d'un ou de plusieurs oxydes d'alkylène au mélange résultant de l'étape d'activation précédente et en attendant ensuite à chaque fois le pic de température ("hotspot") et/ou une chute de pression dans le réacteur, survenant en raison de la réaction chimique exothermique qui suit,

($\gamma$) on ajoute un ou plusieurs oxydes d'alkylène et du dioxyde de carbone au mélange résultant de l'étape (ß) ("copolymérisation").

**8.** Procédé selon l'une quelconque des revendications 1 à 7, **caractérisé en ce que** la copolymérisation d'oxydes d'alkylène et de dioxyde de carbone a lieu sous une pression de 1 à 200 bars.

**9.** Procédé selon l'une quelconque des revendications 1 à 8, **caractérisé en ce que** la copolymérisation d'oxydes d'alkylène et de dioxyde de carbone (étape ($\gamma$)) a lieu dans une plage de température de 60 à 150 °C.

**10.** Procédé selon l'une quelconque des revendications 1 à 9, qui est réalisé dans un réacteur tubulaire, une cuve agitée ou un réacteur à boucle à circulation interne.

**11.** Procédé selon l'une quelconque des revendications 1 à 10 réalisé dans une cuve agitée, **caractérisé en ce que** dans l'étape $\gamma$), un ou plusieurs composés de départ à fonctionnalité H sont dosés en continu dans le réacteur pendant la réaction.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4500704 A
- WO 2006103213 A
- EP 1359177 A
- US 3404109 A
- US 3829505 A
- US 3941849 A
- US 5158922 A
- US 5470813 A
- EP 700949 A
- EP 743093 A
- EP 761708 A
- WO 9740086 A
- WO 9816310 A
- WO 0047649 A
- US 5783513 A
- US 6716788 B2
- JP 4145123 B
- WO 0139883 A
- WO 0180994 A
- WO 2004081082 A


### In der Beschreibung aufgeführte Nicht-Patentliteratur


- **INOUE et al.** Copolymerization of Carbon Dioxide and Epoxide with Organometallic Compounds. *Die Makromolekulare Chemie,* 1969, vol. 130, 210-220
- Handbook of Chemistry and Physics